# EUROPEAN PATENT APPLICATION

(11) **EP 2 332 941 A2**
(43) Date of publication of application: **15.06.2011**
(21) Application number: 09814774.7
(22) Date of filing: 16.09.2009
(51) Int. Cl.: C07D 473/32

(54) **STABILIZED SOLID DISPERSION OF ADEFOVIR DIPIVOXIL AND PREPARATION METHOD THEREOF**

(30) Priority: 17.09.2008 KR 20080090869
(71) Applicant: CJ Cheiljedang Corporation, Jung-gu, Seoul 100-749 (KR)
(72) Inventor: LEE, Yong Tack, Seoul 153-035 (KR); YOON, Myeong Sik, Yongin-si Gyeonggi-do 448-160 (KR); HONG, Hye Suk, Seongnam-si Gyeonggi-do 463-050 (KR); CHO, Il Hwan, Seoul 157-200 (KR); AN, Tae Kun, Yongin-si Gyeonggi-do 449-040 (KR); LEE, Si Beum, Yongin-si Gyeonggi-do 446-753 (KR); JANG, Hae Jong, Seoul 110-030 (KR); PARK, Mi Kyung, Icheon-si Gyeonggi-do 467-812 (KR)
(74) Representative: Nevant, Marc
(86) International application number: PCT/KR2009/005271
(87) International publication number: WO 2010/032958

(57) **Abstract**

The present invention relates to an amorphous solid dispersion with improved stability comprising 9-[2-[[bis[(pivaloyloxy)methyl]phosphono]methoxy]ethyl]adenine as a nucleotide analogue, a pharmaceutical composition comprising the same, and a method for preparing the same.

## Description

### [Technical Field]

The present relates to an amorphous solid dispersion with improved stability, which comprises 9-[2-[[bis[(pivaloyloxy)methyl]phosphono]methoxy]ethyl]adenine (adefovir dipivoxil (compound of Formula 1), or hereinafter, referred to as "ADE") as a nucleotide analogue; and a pharmaceutical composition comprising the same.

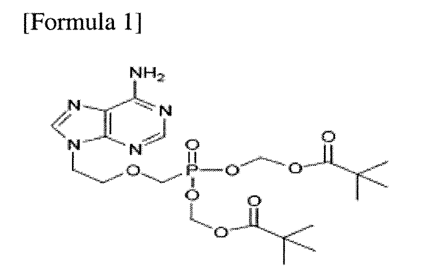

Further, the present invention relates to a method of preparing the amorphous solid dispersion with improved stability, which comprises the steps of dissolving ADE and a water-soluble polymer substance in an organic solvent, and allowing the resulting solution to be adsorbed to a sugar alcohol carrier or dispersed therein using a fluidized bed granulator or a spray dryer.

### [Background Art]

Adefovir dipivoxil (ADE), which is an antiviral drug, is a nucleotide reverse transcriptase inhibitor and exhibits a marked in vivo antiviral activity against especially both HIV and Hepatitis type B virus (HBV). For more information about its antiviral activities, see Starret et al., J. Med. Chem., 37:1857, 1994, and Samira et al., J. Med. Chem., 39:4958,1996.

It has been known that ADE in nature exists in two forms: amorphous and crystal. U.S. Patent No. 6,451,340 and Korean Patent No. 0618663 disclose a pharmaceutical composition comprising anhydrous crystalline ADE and a method of preparing the same. Further, U.S. Patent No. 6,635,278 and Korean Patent No. 0624214 disclose an ADE composition comprising an alkaline excipient to improve stability and a method of preparing the same.

However, it has been found that when ADE is in contact with moisture, ADE is easily and rapidly hydrolyzed and thus byproducts of the hydrolysis accelerate further degradation of ADE *{see,* Yuan et al., Pharm. Res. 17:1098, 2000).

The pharmaceutical composition of crystalline ADE has been sold on the market under the trade designation Hepsera (GSK). However, since a process of preparing the ADE pharmaceutical composition requires water to formulate granules, there is a need to perform a further drying step to reduce the moisture content of the granules to 1.5% or lower. Therefore, it is impossible to satisfy the need for stability because of the contact with moisture for a long time at high temperature.

Further, it has been reported that an ADE solid dispersion is prepared by using a polymer substance, such as hydroxypropyl methylcellulose (HPMC) and polyvinylpyrrolidone (PVP) in combination with an organic solvent. However, the polymer substance adsorbs the surrounding moisture, leading to the acceleration of ADE degradation. Further, since there is a risk of incurring a morphological change of the solid dispersion due to long term exposure at high temperature, the extent of stability improvement is not enough to satisfy.

### [Disclosure]

### [Technical Problem]

Accordingly, an object of the present invention is to provide adefovir dipivoxil in a solid dispersion form with improved stability to heat and moisture as compared with conventional adefovir dipivoxil preparations.

### [Technical Solution]

The present invention relates to an amorphous solid dispersion with improved stability, which comprises ADE of Formula 1, a water-soluble polymer substance and a sugar alcohol, and a pharmaceutical composition comprising the same for the treatment of HIV infection or hepatitis B.

Also, the present invention relates to a method of preparing an amorphous solid dispersion with solubility and improved stability, which comprises the steps of dissolving a water-soluble polymer substance and adefovir dipivoxil in an organic solvent, e.g., acetone, or a mixed solution of acetone and a pharmaceutically acceptable organic solvent; and allowing the resulting solution to be adsorbed to a sugar alcohol carrier or dispersed therein.

A preferred water-soluble polymer according to the present invention is one or more selected from the group consisting of polyethyleneglycol (PEG), polyvinylalcohol (PVA), hydroxypropyl methylcellulose (HPMC), and polyvinylpyrrolidone (PVP). A more preferable water-soluble polymer is a vinylpyrrolidone-vinylacetate copolymer (Kollidone VA64). U.S. Patent No. 4,301,146 discloses a stabilized solid dispersion of a drug which is made only of a polymer substance. In this case, the polymer substances can prevent drug molecules from contacting with water molecules, thereby improving its stability. However, such a polymer substance tends to easily absorb moisture, thereby decreasing the stability of most drug.

In the solid dispersion according to the present invention, the weight ratio of ADE to the water-soluble polymer is preferably from 1:0.1 to 1:10, more preferably from 1:0.5 to 1:5. If the weight ratio of the water-soluble polymer is lower than 0.1 with respect to 1 part by weight of ADE, it is difficult to prepare an amorphous solid dispersion, while if that exceeds 10, it is unsuitable to formulate it into a tablet.

Further, the weight ratio of ADE to the sugar alcohol is preferably from 1:0.5 to 1:10, more preferably from 1:3 to 1:6. If the weight ratio of the sugar alcohol is lower than 0.5 with respect to 1 part by weight of ADE, the stability of a solid dispersion is decreased, while that exceeds 10, it is difficult to formulate.

The sugar alcohol according to the present invention is one or more selected from the group consisting of lactose, glucose, mannitol, sorbitol, and isomalt, wherein lactose or isomalt is more preferred.

It has been reported that if a certain drug is compressed together with sugar alcohol having binding activity, pharmaceutical uses of the compressed drug formulation can be improved. Since most sugar alcohols are hydroscopic, the larger their surface area becomes by pulverization, the more the formulation adsorbs the surrounding moisture, thereby decreasing the stability. However, when the sugar alcohol is pulverized and sufficiently dispersed between respective particles, the compressed formulation can maintain its stability in terms of pulverizing strength and moisture adsorption. Representative examples of such a sugar alcohol may include sorbitol, isomalt and the like.

The present invention relates to a pharmaceutical composition further comprising pharmaceutically acceptable carriers in addition to the amorphous solid dispersion of adefovir dipivoxil. The pharmaceutically acceptable carriers may be one or more selected from the group consisting of diluents, disintegrating agents, binding agents, and lubricants. The carrier may make it possible to regulate the dissolution rate and disintegration time of the solid dispersion, leading to the control of bioavailability and improvement in stability of the solid dispersion. Suitable excipients may include starch, sucrose, microcrystalline cellulose, dibasic sodium phosphate, monobasic potassium phosphate, maltodextrin, dextrin, cyclodextrin, galactose and the like.

### [Advantageous Effects]

The present invention provides a solid dispersion which is less sensitive to heat and moisture and thus is thermodynamically more stable as compared with a conventional solid dispersion, and a pharmaceutical composition comprising the same.

### [Description of Drawings]

Fig. 1 is a XRD pattern of crystalline ADE which shows characteristic peaks represented by 2θ at approximately 7.4, 7.9, 10.2, 12.4, 15.1, 16.4, 17.3, 18.0, 20.2, 21.4, and 22.3.
Fig. 2 is a XRD pattern of an amorphous solid dispersion of Comparative Example 1.
Fig. 3 is a XRD pattern of isomalt used as a sugar alcohol.
Fig. 4 is a XRD pattern of an amorphous solid dispersion of Example 6 wherein a characteristic peak of ADE is not observed and there is only an XRD pattern of isomalt, suggesting that ADE exists in an amorphous form.

### [Mode for Invention]

Hereinafter, the present invention will be described in detail with in the following examples. However, the examples of the present invention are only for illustrative purposes and are not construed as being limited to the scope of the present invention.

### [Example 1]

10 g of adefovir dipivoxil and 30 g of Kollidone VA64 were completely dissolved in 100 g of acetone. The solution was spray dried with 60 g of lactose as a carrier by using a fluidized bed granulator (Glatt GPCG-1, Germany), to obtain an amorphous adefovir dipivoxil solid dispersion. Here, an inflow temperature of the fluidized bed drying was 65°C, and a discharge temperature thereof was in a range of 35 to 45°C.

### [Example 2]

An amorphous adefovir dipivoxil solid dispersion was prepared according to the same method as described in Example 1 except that 30 g of lactose was used.

### [Example 3]

An amorphous adefovir dipivoxil solid dispersion was prepared according to the same method as described in Example 1 except that 90 g of lactose was used.

### [Example 4]

An amorphous adefovir dipivoxil solid dispersion was prepared according to the same method as described in Example 1 except that 15 g of Kollidone VA64 was used.

### [Example 5]

An amorphous adefovir dipivoxil solid dispersion was prepared according to the same method as described in Example 1 except that 20 g of Kollidone VA64 was used.

### [Example 6]

An amorphous adefovir dipivoxil solid dispersion was prepared according to the same method as described in Example 1 except that 60 g of isomalt was used as a carrier instead of lactose.

### [Example 7]

An amorphous adefovir dipivoxil solid dispersion was prepared according to the same method as described in Example 1 except that 30 g of isomalt was used as a carrier instead of lactose.

### [Example 8]

An amorphous adefovir dipivoxil solid dispersion was prepared according to the same method as described in Example 1 except that 200 ml of ethanol was used as an organic solvent instead of 100 ml of acetone.

### [Example 9]

An amorphous adefovir dipivoxil solid dispersion was prepared according to the same method as described in Example 1 except that 200 ml of methanol was used as an organic solvent instead of 100 ml of acetone.

### [Example 10]

An amorphous adefovir dipivoxil solid dispersion was prepared according to the same method as described in Example 1 except that methylene chloride/methanol (50 ml/50 ml) was used as an organic solvent instead of 100 ml of acetone.

### [Comparative Example 1] Preparation of solid dispersion comprising adefovir dipivoxil and Kollidone VA64

10 g of adefovir dipivoxil and 30 g of Kollidone VA64 were completely dissolved in 100 g of acetone. The solution was spray dried by using a spray dryer (Büchi Mini Spray Dryer, B-191, Switzerland), to obtain an amorphous adefovir dipivoxil solid dispersion. Here, the spray drying was carried out under the same conditions as described in Example 1.

### [Comparative Example 2]

An amorphous adefovir dipivoxil solid dispersion was prepared according to the same method as described in Comparative Example 1 except that 10 g of Kollidone VA64 was used.

### [Comparative Example 3]

An amorphous adefovir dipivoxil solid dispersion was prepared according to the same method as described in Comparative Example 1 except that 50 g of Kollidone VA64 was used.

### [Test Example 1] X-ray powder diffraction analysis of amorphous adefovir dipivoxil

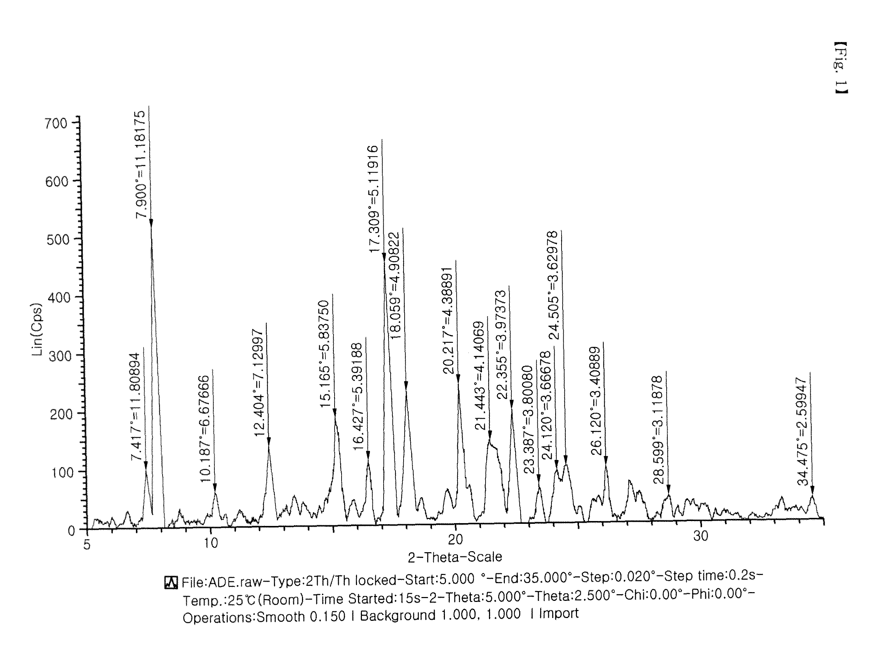

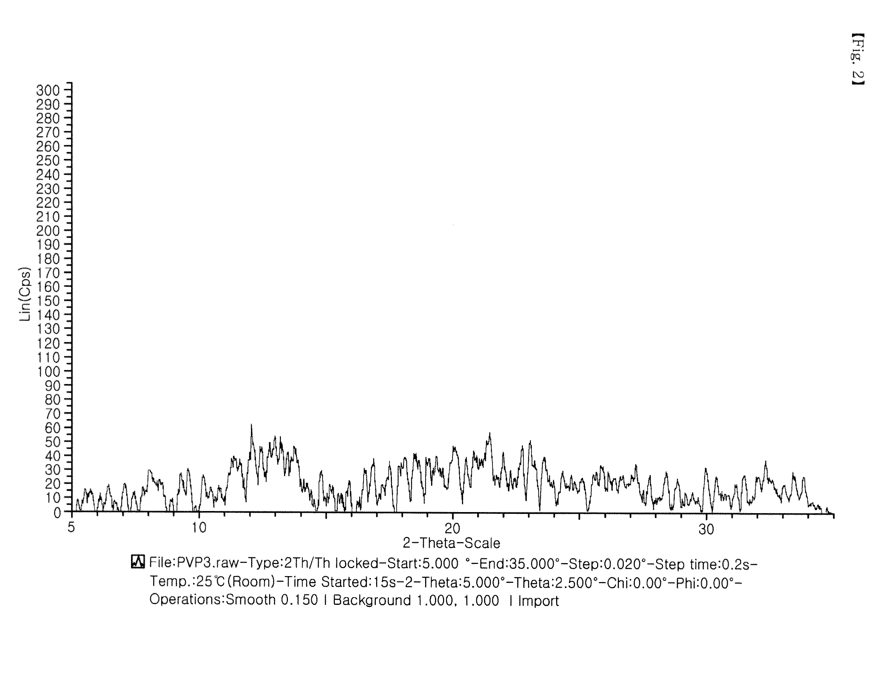

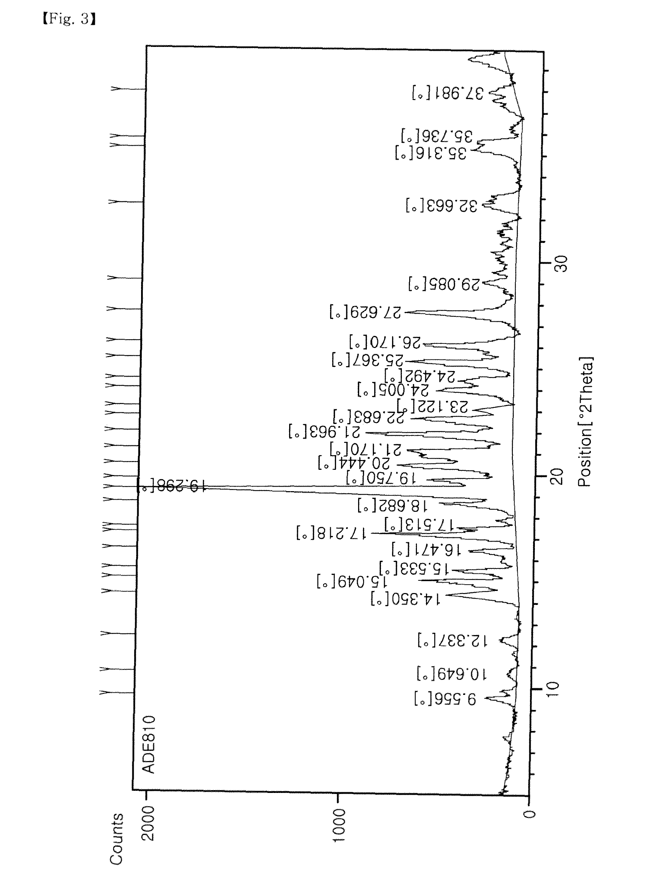

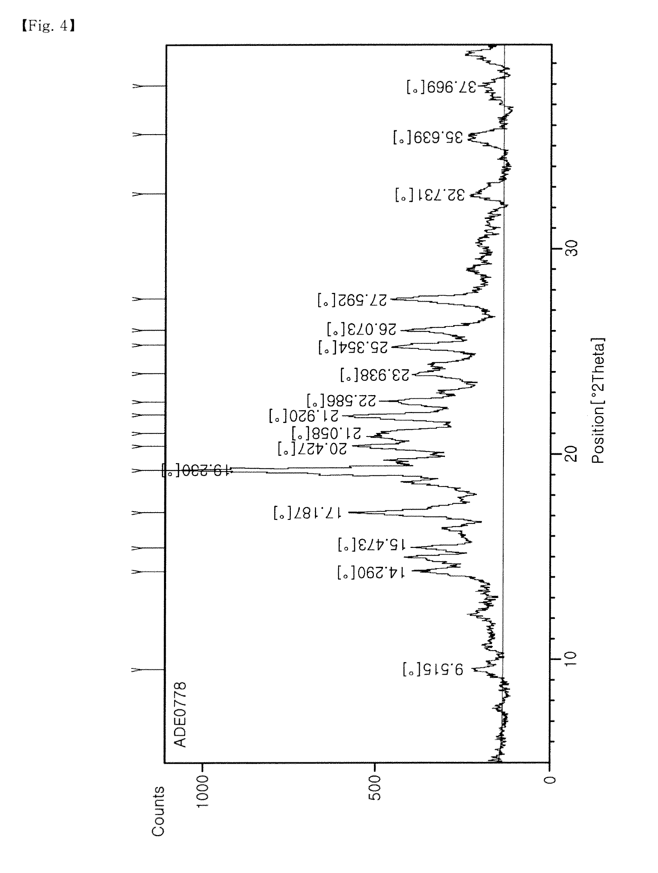

Crystalline adefovir dipivoxil, the amorphous solid dispersion of Comparative Example 1, isomalt as a sugar alcohol, and the amorphous solid dispersion of Example 6 were packaged into inducible closed type high-density polyethylene containers under conditions of 60°C, relative humidity of 75%, respectively. Four weeks after, each sample was subjected to X-ray powder diffraction analysis by using X-ray diffractometer (model: X'pert-pro), and the results are shown in Figs. 1 to 4.

### [Test Example 2] Thermodynamic stability test of amorphous adefovir dipivoxil

The amorphous solid dispersion was packaged together with 3 g of silica gel into an inducible closed type high-density polyethylene container under conditions of 40°C, relative humidity of 75%. Residual ADE content was measured according to an area normalization method at different times. Table 1 represents the degree of ADE degradation depending on the addition of sugar alcohol.

**Table 1**

| | Residual ADE content | | | |
|---|---|---|---|---|
| | 0-week | 1-week | 2-week | 4-week |
| Example 1 | 99.64 | 99.25 | 98.9 | 98.14 |
| Example 6 | 99.57 | - | 99.23 | 98.47 |
| Comparative Example 1 | 99.01 | 98.01 | 97.29 | 94.14 |

### [Preparation Example 1] Preparation of tablet

100 g of the amorphous adefovir dipivoxil solid dispersion (10 g as adefovir dipivoxil) of Example 1, 23 g of lactose, 7.5 g of pre-gelatinized starch, 12 g of croscarmellose sodium, 9 g of talc and 1.5 g of magnesium stearate were homogeneously mixed and formulated into a tablet containing 10 mg of adefovir dipivoxil per tablet.

### [Preparation Example 2] Preparation of tablet

A tablet was prepared according to the same method as described in Preparation Example 1 except that the amorphous adefovir dipivoxil solid dispersion of Example 6 was used.

## Claims

1. An amorphous adefovir dipivoxil solid dispersion, comprising:
adefovir dipivoxil;
a water-soluble polymer substance; and
a sugar alcohol.

2. The amorphous adefovir dipivoxil solid dispersion as claimed in Claim 1, wherein the weight ratio of adefovir dipivoxil to the water-soluble polymer substance is from 1:0.1 to 1:10.

3. The amorphous adefovir dipivoxil solid dispersion as claimed in Claim 1, wherein the weight ratio of adefovir dipivoxil to the sugar alcohol is from 1:0.5 to 1:10.

4. The amorphous adefovir dipivoxil solid dispersion as claimed in Claim 1, wherein the water-soluble polymer substance is one or more selected from the group consisting of hydroxymethylcellulose, polyvinylpyrrolidone, hydroxypropylcellulose, polyethyleneglycol, polyvinylalcohol, and vinylpyrrolidone/vinylacetate copolymer.

5. The amorphous adefovir dipivoxil solid dispersion as claimed in any one of Claims 1 to 4, wherein the sugar alcohol is one or more selected from the group consisting of lactose, glucose, mannitol, sorbitol and isomalt.

6. A pharmaceutical composition for treating HIV infection or hepatitis B, comprising:
the solid dispersion according to Claim 5; and
a pharmaceutically acceptable carrier.

7. A method of preparing the amorphous adefovir dipivoxil solid dispersion according to any one of Claims 1 to 4, comprising the steps of:
dissolving the water-soluble polymer substance and adefovir dipivoxil in an organic solvent; and
allowing the resulting solution to be adsorbed to the sugar alcohol or dispersed therein.

8. The method as claimed in Claim 7, wherein the sugar alcohol is one or more selected from the group consisting of lactose, glucose, mannitol, sorbitol, and isomalt.
